(19) Europäisches Patentamt
European Patent Office
Office européen des brevets

(11) Numéro de publication: **0 511 087 B1**

(12) # FASCICULE DE BREVET EUROPEEN

(45) Date de publication de fascicule du brevet: **28.06.95**

(51) Int. Cl.[6]: **A23L 1/236**, C07D 213/84

(21) Numéro de dépôt: **92401129.9**

(22) Date de dépôt: **22.04.92**

(54) **Agents édulcorants dérivant de l'acide l-glutamique; leur procédé de préparation.**

(30) Priorité: **23.04.91 FR 9105001**

(43) Date de publication de la demande:
**28.10.92 Bulletin 92/44**

(45) Mention de la délivrance du brevet:
**28.06.95 Bulletin 95/26**

(84) Etats contractants désignés:
**AT BE CH DE DK ES FR GB GR IT LI LU MC NL PT SE**

(56) Documents cités:
**EP-A- 0 338 946**
**WO-A-91/06224**

**PATENT ABSTRACTS OF JAPAN vol. 12, no. 132 (C-490)22 Avril 1988**

(73) Titulaire: **NOFRE, Claude**
**119, Cours Albert Thomas**
**F-69003 Lyon (FR)**

Titulaire: **TINTI, Jean-Marie**
**5, impasse de la Drelatière**
**F-69680 Chassieu (FR)**

(72) Inventeur: **NOFRE, Claude**
**119, Cours Albert Thomas**
**F-69003 Lyon (FR)**
Inventeur: **TINTI, Jean-Marie**
**5, impasse de la Drelatière**
**F-69680 Chassieu (FR)**

(74) Mandataire: **Hubert, Philippe et al**
**Cabinet Beau de Loménie**
**158, rue de l'Université**
**F-75340 Paris Cédex 07 (FR)**

EP 0 511 087 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouveaux agents édulcorants dérivant de l'acide L-glutamique, ainsi que leur procédé de préparation.

Ces nouveaux agents édulcorants sont particulièrement utiles pour édulcorer des produits variés, et en particulier les boissons, notamment les boissons gazeuses, les aliments, les confiseries, les patisseries, les chewing-gums, les produits d'hygiène et les articles de toilette, ainsi que les produits cosmétiques, pharmaceutiques et vétérinaires.

On sait que, pour être utilisable à l'échelle industrielle, un agent édulcorant doit posséder d'une part un pouvoir sucrant intense, permettant de limiter le coût d'utilisation, et d'autre part, une stabilité satisfaisante, c'est-à-dire compatible avec les conditions d'utilisation.

Dans le cas particulier des boissons gazeuses, qui représentent l'utilisation principale des agents édulcorants, il est très difficile d'obtenir une stabilité satisfaisante, d'autant plus que certaines de ces boissons ont la particularité d'être acides avec un pH généralement compris entre 2,5 et 3,5.

Le document JP-A-87-252754 révèle en termes généraux des édulcorants de formule générale :

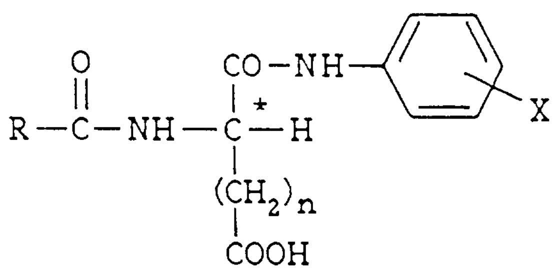

dans laquelle X est CN ou $NO_2$, R est H, ou un groupe alkyle, aryle ou alcoxy en $C_1$-$C_{10}$, ou aryloxy, n est égal à 1 ou 2. Le groupe marqué avec l'astérisque indique une configuration L, D ou DL de l'amino acide.

Sur les 15 exemples spécifiquement décrits, 14 composés sont des dérivés de l'acide aspartique (n = 1) et un seul est un dérivé de l'acide glutamique (n = 2). Par ailleurs, dans ces composés, le substituant X est toujours en position para. Le pouvoir sucrant (SP) de ces composés connus (par comparaison avec une solution de saccharose à 5 %) est compris entre 2 et 720 fois celui du saccharose (Tableau 1).

TABLEAU 1

| R | * | n | X | SP |
|---|---|---|---|---|
| H | D | 1 | CN | 110 |
| H | D | 1 | $NO_2$ | 50 |
| $CH_3O$ | L | 1 | CN | 70 |
| $CH_3O$ | D | 1 | CN | 140 |
| $C_2H_5O$ | L | 1 | CN | 80 |
| $C_6H_5O$ | L | 1 | CN | 90 |
| $C_6H_5CH_2O$ | L | 1 | CN | 260 |
| $C_6H_5CH_2O$ | D | 1 | CN | 110 |
| $C_6H_5$ | L | 1 | CN | 720 |
| $CH_3$ | D | 1 | CN | 10 |
| $C_6H_5CH_2O$ | D | 1 | $NO_2$ | 70 |
| $C_6H_5$ | L | 1 | $NO_2$ | 420 |
| $C_6H_5CH_2O$ | L | 2 | CN | 2 |
| H | L | 1 | CN | 40 |
| H | L | 1 | $NO_2$ | 2 |

Parmi ceux-ci, le composé possédant le pouvoir édulcorant le plus élevé (720 fois celui du saccharose) dérive de l'acide L-aspartique, et répond à la formule (1) :

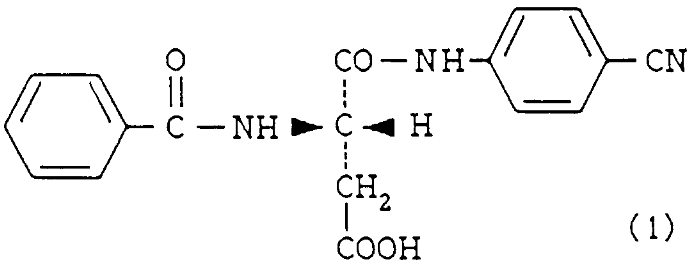

(1)

Le seul composé décrit dérivant de l'acide L-glutamique répond à la formule (2) :

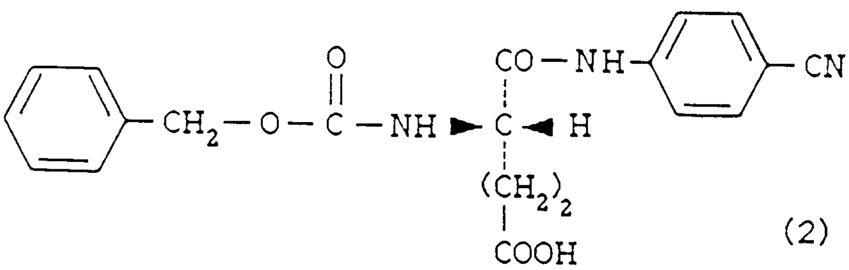

(2)

Ce composé possède un pouvoir sucrant très faible, 2 fois celui du saccharose, ce qui en exclut toute possibilité d'application industrielle.

Dans le document PCT/FR 90/00765 des Demandeurs, il a été décrit des composés édulcorants beaucoup plus puissants et beaucoup plus stables que ceux cités dans le document JP-A-87-252 754.

Ces composés répondent à la formule générale suivante :

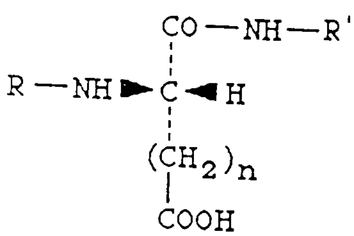

dans laquelle :

R est un groupe acyle de formule :

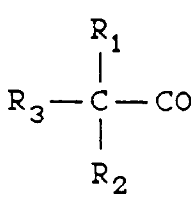

dans laquelle :

$R_1$ est un radical méthyle, éthyle, propyle, isopropyle, phényle, méthoxy, éthoxy, trihalogénométhyle, chloro

ou chlorométhyle ;

$R_2$ est un atome d'hydrogène ou un radical méthyle, éthyle ou méthoxy ;

ou bien dans laquelle $R_1$ et $R_2$ pris ensemble avec l'atome de carbone auquel ils sont liés forment un groupe cycloalkyle ayant de 3 à 6 atomes de carbone;

$R_3$ est un radical alkyle ayant de 3 à 11 atomes de carbone, un radical alcényle ayant de 3 à 7 atomes de carbone, un radical cycloalkyle ayant de 3 à 7 atomes de carbone, un radical cycloalkylalkyle dont la partie cycloalkyle présente de 3 à 6 atomes de carbone et la partie alkyle présente de 1 à 3 atomes de carbone, un radical phényle, un radical phénylalkyle dont la partie alkyle présente de 1 à 3 atomes de carbone, un radical alcoxy ayant de 3 à 10 atomes de carbone, un radical cycloalkyloxy ayant de 3 à 6 atomes de carbone et dont les deux positions adjacentes au carbone 1 fixé à l'oxygène peuvent chacune être substituées par 1 ou 2 groupes méthyle, un radical cycloalkylalcoxy dont la partie cycloalkyle présente de 3 à 6 atomes de carbone et la partie alcoxy présente de 1 à 3 atomes de carbone, un radical phénoxy, un radical phénylalcoxy dont la partie alcoxy présente de 1 à 3 atomes de carbone ;

n est égal à 1 ou 2 ;

R' est un groupe de formule :

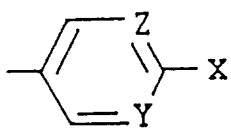

dans laquelle Y et Z, identiques ou différents, représentent N ou CH, et dans laquelle X est choisi dans le groupe constitué par CN, $NO_2$, Cl, $CF_3$, $COOCH_3$, $COCH_3$, $COCF_3$, $CONH_2$, $CON(CH_3)_2$, $SO_2CH_3$, $N_3$ ou H ; ainsi que ses sels physiologiquement acceptables.

Dans le document PCT/FR 90/00765, 44 composés sont décrits et le pouvoir sucrant de ceux-ci peut atteindre 25 000 fois celui du saccharose (Tableau 2).

## TABLEAU 2

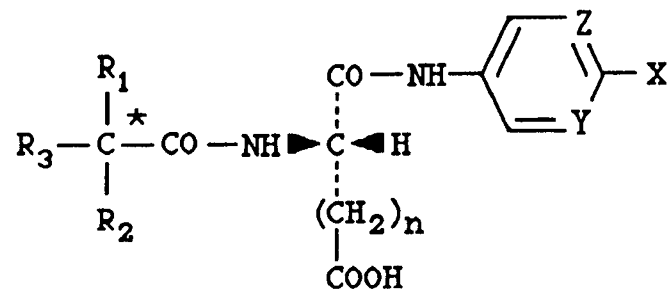

| $R_1$ | $R_2$ | $R_3$ | * | n | Y | Z | X | SP |
|---|---|---|---|---|---|---|---|---|
| $CH_3$ | H | $CH_3(CH_2)_2$ | R | 2 | CH | CH | CN | 500 |
| $CH_3$ | H | $CH_3(CH_2)_2$ | S | 2 | CH | CH | CN | 2800 |
| $CH_3$ | H | $CH_3(CH_2)_3$ | R | 2 | CH | CH | CN | 3000 |
| $CH_3$ | H | $CH_3(CH_2)_3$ | S | 2 | CH | CH | CN | 9000 |
| $CH_3$ | H | $(CH_3)_2CH(CH_2)_2$ | RS | 2 | CH | CH | CN | 6000 |
| $CH_3$ | H | $CH_3(CH_2)_4$ | RS | 2 | CH | CH | CN | 1000 |
| $CH_3$ | H | $C_6H_5$ | R | 2 | CH | CH | CN | 1300 |
| $CH_3$ | H | $C_6H_5$ | S | 2 | CH | CH | CN | 1500 |
| $CH_3$ | H | $CH_3(CH_2)_3$ | R | 2 | N | CH | CN | 2000 |
| $CH_3$ | H | $CH_3(CH_2)_3$ | S | 2 | N | CH | CN | 20000 |
| $CH_3$ | H | $C_6H_5$ | R | 2 | N | CH | CN | 2500 |

TABLEAU 2 (suite)

| $R_1$ | $R_2$ | $R_3$ | * | n | Y | Z | X | SP |
|---|---|---|---|---|---|---|---|---|
| $CH_3$ | H | $C_6H_5$ | S | 2 | N | CH | CN | 4000 |
| $CH_3$ | H | $c-C_6H_{11}$ | RS | 2 | CH | CH | CN | 1000 |
| $C_2H_5$ | H | $CH_3(CH_2)_3$ | RS | 2 | CH | CH | CN | 5000 |
| $C_2H_5$ | H | $C_6H_5$ | RS | 2 | CH | CH | CN | 2700 |
| $C_6H_5$ | H | $C_6H_5$ |  | 2 | CH | CH | CN | 250 |
| $CH_3O$ | H | $C_6H_5$ | RS | 2 | CH | CH | CN | 2300 |
| $CH_3O$ | H | $C_6H_5$ | R | 2 | N | CH | CN | 11000 |
| $CH_3O$ | H | $C_6H_5$ | S | 2 | N | CH | CN | 3000 |
| $CH_3O$ | H | $CH_3(CH_2)_3$ | RS | 2 | N | CH | CN | 2000 |
| $CH_3$ | H | $C_6H_5O$ | RS | 2 | CH | CH | CN | 4000 |
| $CH_3$ | H | $CH_3(CH_2)_2O$ | R | 2 | CH | CH | CN | 400 |
| $CH_3$ | H | $CH_3(CH_2)_2O$ | S | 2 | CH | CH | CN | 200 |
| $CH_3$ | H | (2,6-diMe)-c-$C_6H_9O$ | RS | 2 | CH | CH | CN | 1000 |
| $CH_3$ | H | $C_6H_5O$ | RS | 2 | N | CH | CN | 13000 |
| $CH_3$ | H | $C_6H_5O$ | R | 2 | N | CH | CN | 25000 |
| Cl | H | $C_6H_5$ | RS | 2 | CH | CH | CN | 2200 |

**TABLEAU 2 (suite)**

| $R_1$ | $R_2$ | $R_3$ | * | n | Y | Z | X | SP |
|---|---|---|---|---|---|---|---|---|
| $CF_3$ | $CH_3O$ | $C_6H_5$ | S | 2 | CH | CH | CN | 600 |
| $CH_3$ | $CH_3$ | $CH_2{=}CHCH_2$ | | 2 | CH | CH | CN | 500 |
| $CH_3$ | $CH_3$ | $CH_3(CH_2)_3$ | | 2 | CH | CH | CN | 11000 |
| $CH_3$ | $CH_3$ | $(CH_3)_2CH(CH_2)_2$ | | 2 | CH | CH | CN | 10000 |
| $CH_3$ | $CH_3$ | $(CH_3)_3CH(CH_2)_2$ | | 2 | CH | CH | CN | 4000 |
| $CH_3$ | $CH_3$ | $CH_3(CH_2)_3$ | | 2 | N | CH | CN | 22000 |
| $CH_3$ | $CH_3$ | $CH_3(CH_2)_4$ | | 2 | N | CH | CN | 3000 |
| $CH_2-CH_2$ | | $C_6H_5$ | | 2 | CH | CH | CN | 2500 |
| $CH_2(CH_2)_2CH_2$ | | $C_6H_5$ | | 2 | CH | CH | CN | 2000 |
| $CH_2(CH_2)_2CH_2$ | | $C_6H_5$ | | 2 | N | CH | CN | 3000 |
| $CH_2-CH_2$ | | $C_6H_5$ | | 1 | CH | CH | CN | 1000 |
| $CH_3$ | H | $CH_3(CH_2)_3$ | RS | 1 | CH | CH | CN | 1500 |
| $CH_3$ | H | $C_6H_5O$ | R | 1 | CH | CH | CN | 18000 |
| $CH_3$ | H | $CH_3(CH_2)_3$ | S | 2 | CH | CH | $COCH_3$ | 300 |
| $CH_3$ | H | $CH_3(CH_2)_3$ | S | 2 | CH | CH | $CONH_2$ | 700 |
| $CH_3$ | $CH_3$ | $CH_3(CH_2)_3$ | | 2 | N | CH | Cl | 600 |
| $CH_3$ | H | $CH_3(CH_2)_3$ | S | 2 | N | N | CN | 10000 |

Les composés décrits à titre préférentiel dans ce document PCT/FR 90/00765 sont représentés par les formules (3) (4) et (5) ci-après :

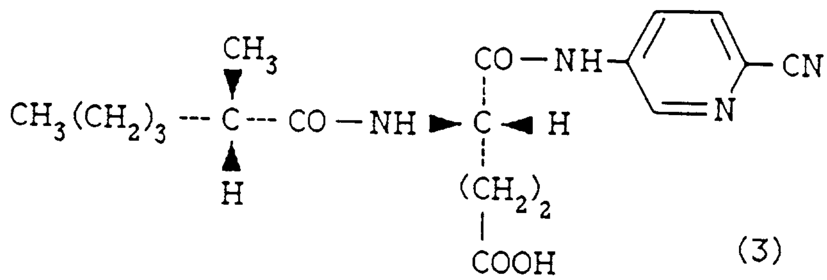

(3)

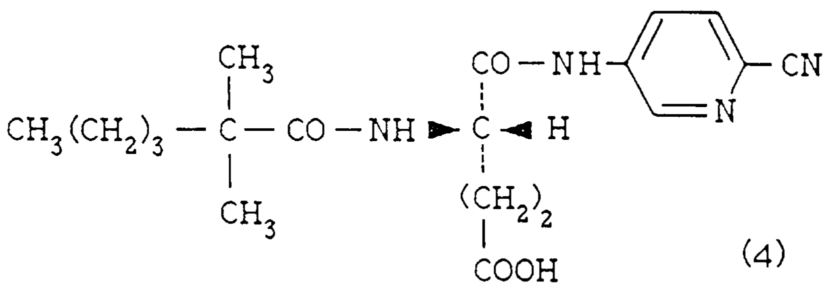

(4)

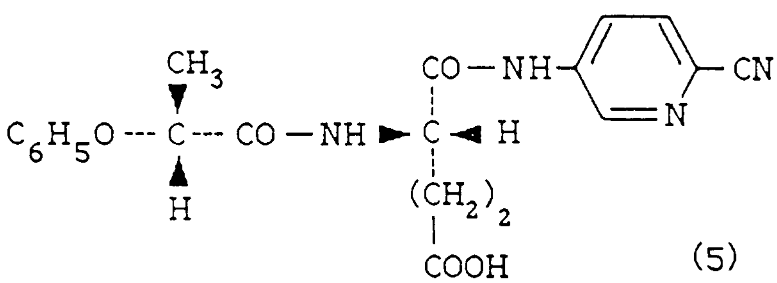

(5)

Le composé de formule (3) a un pouvoir sucrant de 20 000 fois celui du saccharose (par rapport à une solution de saccharose à 2 %) et une stabilité évaluée par sa demi-vie d'environ 60 jours à 70 °C et à pH 3.

Le composé de formule (4) a un pouvoir sucrant de 22 000 fois celui du saccharose et une demi-vie d'environ 70 jours à pH 3 et à 70 °C.

Le composé de formule (5) a un pouvoir sucrant de 25 000 fois celui du saccharose et une demi-vie d'environ 60 jours à pH 3 et 70 °C.

Ainsi, les composés préférentiels selon ce document antérieur peuvent être représentés par la formule générale suivante :

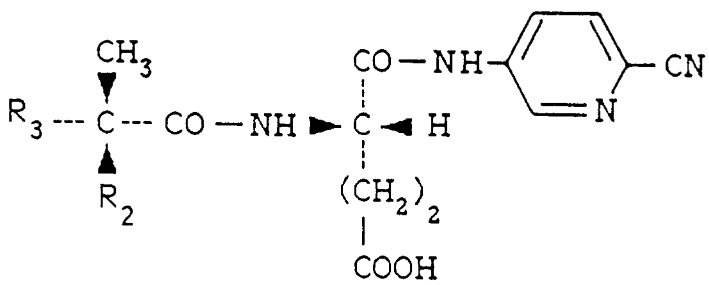

dans laquelle $R_2$ est H ou $CH_3$, et $R_3$ est $C_4H_9$ quand $R_2$ est H ou $CH_3$, et $C_6H_5O$ quand $R_2$ est H.

Ces composés se caractérisent par des pouvoirs sucrants élevés compris entre 20 000 et 25 000 et par une grande stabilité (demi-vie à pH 3 et à 70 °C d'environ 60 à 70 jours).

Le présente invention a pour but de fournir de nouveaux agents édulcorants, dérivés de l'acide L-glutamique, encore plus puissants et plus stables que ceux décrits dans l'art antérieur.

Ces agents édulcorants répondent à la formule générale suivante :

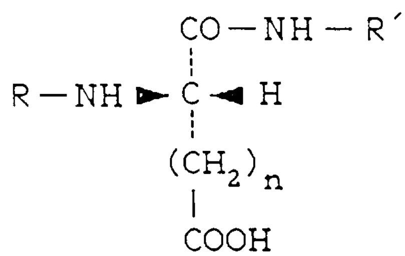

dans laquelle R est un radical 5,6,7,8-tétrahydro-1-naphtoyle de formule :

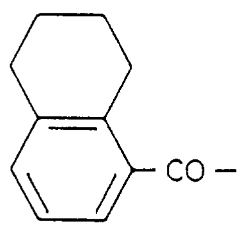

ou un radical 1-naphtoyle de formule :

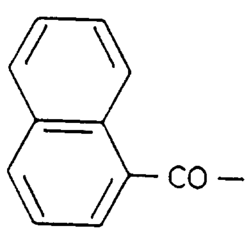

dans laquelle R' est un radical 2-cyanopyrid-5-yle de formule :

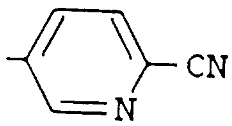

et dans laquelle n est égal à 2.

En d'autres termes, l'invention vise à couvrir les deux composés de formules (6) et (7) suivantes :

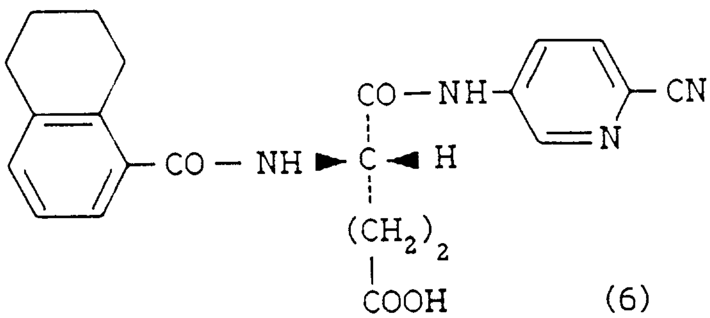

(6)

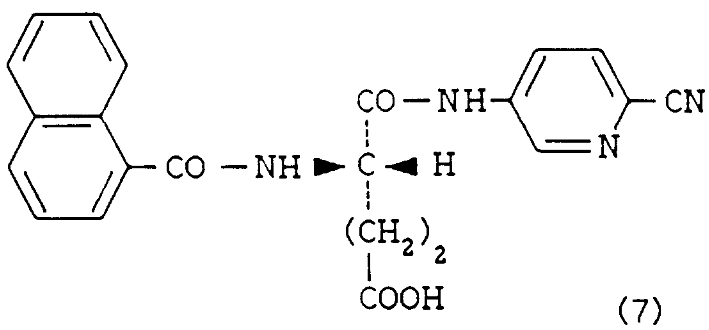

(7)

Le composé de formule (6) a un pouvoir sucrant, sur une base pondérale, de 40 000 fois celui du saccharose (par rapport à une solution de saccharose à 2 %) et une stabilité, évaluée par sa demi-vie à pH 3 et à 70 °C, qui est de l'ordre de 100 jours.

Le composé de formule (7) a un pouvoir sucrant, sur une base pondérale, de 30 000 fois celui du saccharose (par rapport à une solution de saccharose à 2 %) et une stabilité, évaluée par sa demi-vie à pH 3 et à 70 °C, qui est aussi de l'ordre de 100 jours.

L'invention résulte donc de la sélection dans l'état de la technique de deux composés dérivant de l'acide L-glutamique dont les substituants R et R' spécifiquement choisis permettent de façon tout à fait inattendue d'obtenir pour ces composés un pouvoir sucrant beaucoup plus intense que ceux des composés décrits dans l'art antérieur, ce qui permet de baisser de façon notable le prix de revient de ces composés et donc leur coût d'utilisation, d'autant plus que le groupe acyle R qui est formé d'un groupe bicyclique ortho-fusionné ne possède pas de centre chiral, comme c'est le cas pour les composés 3 et 5 de l'art antérieur. Les composés de l'invention ont de plus une grande stabilité en solution acide, beaucoup plus importante que celle des composés décrits dans l'art antérieur, ce qui permet de répondre parfaitement aux exigences pratiques qu'impose l'utilisation d'édulcorants, notamment pour les boissons gazeuses (pH 3).

Pour démontrer les avantages des composés de l'invention par rapport aux composés de l'art antérieur, le pouvoir sucrant (SP) et la stabilité (évaluée par la demi-vie $t_{0.5}$ à pH 3 et à 70 °C) des principaux composés de l'art antérieur (composés 1 à 5) ont été comparés aux composés de la présente invention (composés 6 et 7).

Les résultats obtenus ont été regroupés au Tableau 3.

TABLEAU 3

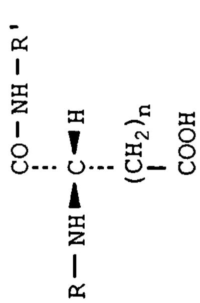

| No. | R | n | R' | SP | $t_{0.5}$ (pH 3, 70 °C) |
|---|---|---|---|---|---|
| 1 | 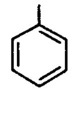 CO- | 1 | 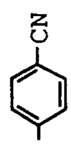 | 720 | 0.33 |
| 2 | 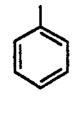 $CH_2OCO-$ | 2 | 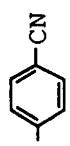 | 2 | 15 |
| 3 | $CH_3(CH_2)_3CH(CH_3)CO-$ | 2 | 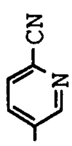 | 20 000 | 60 |
| 4 | $CH_3(CH_2)_3C(CH_3)_2CO-$ | 2 | 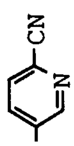 | 22 000 | 70 |
| 5 | 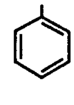 $OCH(CH_3)CO-$ | 2 | 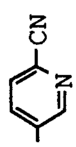 | 25 000 | 60 |
| 6 | 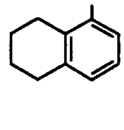 CO- | 2 | 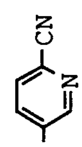 | 40 000 | 100 |
| 7 | 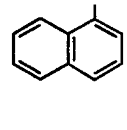 CO- | 2 | 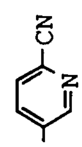 | 30 000 | 100 |

Comme il ressort de ce Tableau, les composés 6 et 7 de la présente invention ont un pouvoir sucrant qui est environ 30 à 40 fois plus élevé que celui du composé 1, qui est le composé le plus puissant à base d'acide L-aspartique (n = 1) décrit dans le document JP-A-87-252754, et ils ont un pouvoir sucrant qui est environ 11 000 à 15 000 fois plus élevé que celui du composé 2, qui est le seul composé à base d'acide L-glutamique (n = 2) décrit dans ce même document (comparaisons effectuées sur la base des pouvoirs sucrants évalués par rapport à une solution de saccharose à 5 %). Enfin, les composés 6 et 7 de la

présente invention ont un pouvoir sucrant qui est environ 1,2 à 1,6 fois plus élevé que celui du composé 5, qui est le composé le plus puissant décrit dans le document PCT/FR 90/00765.

Comme il ressort aussi de ce Tableau, les composés 6 et 7 de la présente invention ont une stabilité qui est environ 300 fois plus grande que celle du composé 1 du document JP-A-87-252754, environ 6 fois plus grande que celle du composé 2 du même document, environ 1,6 fois plus grande que celle du composé 5 du document PCT/FR 90/00765.

Enfin, il est à noter que les composés de l'invention se révèlent beaucoup plus puissants et beaucoup plus stables que ne l'est l'aspartame (formule 8) qui est l'édulcorant de synthèse le plus utilisé.

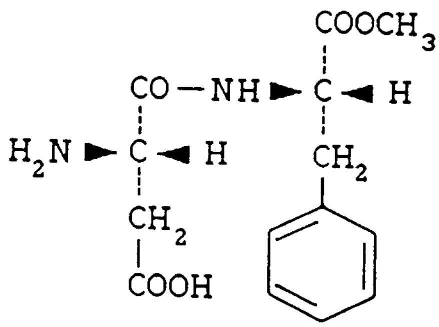

(8)

Le pouvoir sucrant de l'aspartame est en effet 180 fois celui du saccharose et sa stabilité, évaluée par sa demi-vie, est de 1 jour à pH 3 et à 70 °C, ce qui revient à dire que les composés 6 et 7 de la présente invention sont environ 160 à 220 fois plus puissants et environ 100 fois plus stables que l'aspartame.

Les composés 6 et 7 de l'invention sont donc, parmi les composés dérivant de l'acide L-glutamique, les composés les plus puissants et les plus stables jusqu'ici décrits. Ces composés cumulent donc deux propriétés essentielles exigées d'un bon édulcorant, à savoir un très haut pouvoir sucrant et une stabilité très élevée que l'on ne retrouve ni dans les composés de l'art antérieur ni dans l'aspartame.

En ce qui concerne la stabilité, la supériorité des composés de l'invention ressort fort bien du diagramme donné dans la Figure 1 où sont données les courbes de dégradation dans le temps, à pH 3 et à 70 °C, des composés de l'art antérieur (courbes 1 à 5), des composés de la présente invention (courbes 6 et 7), et de l'aspartame (courbe 8).

Les agents édulcorants de la présente invention peuvent être ajoutés à tout produit comestible dans lequel on désire apporter un goût sucré, à condition de les ajouter en proportions suffisantes pour atteindre le niveau de sucrosité désiré. Le concentration optimale d'utilisation de l'agent édulcorant dépendra de facteurs divers tels que, par exemple, le pouvoir sucrant de l'agent édulcorant, les conditions de stockage et d'utilisation des produits, les constituants particuliers des produits, le profil gustatif des produits comestibles et le niveau de sucrosité désiré. Toute personne qualifiée peut facilement déterminer la proportion optimale d'agent édulcorant qui doit être employée pour l'obtention d'un produit comestible en réalisant des analyses sensorielles de routine. Les agents édulcorants de la présente invention sont, en général, ajoutés aux produits comestibles dans des proportions allant, suivant le pouvoir édulcorant du composé, de 0,5 mg à 10 mg d'agent édulcorant par kilogramme ou par litre de produit comestible. Les produits concentrés contiendront évidemment des quantités plus élevées d'agent édulcorant, et seront ensuite dilués suivant les intentions finales d'utilisation.

Les agents édulcorants de la présente invention peuvent être ajoutés sous forme pure aux produits à édulcorer, mais en raison de leur pouvoir sucrant élevé, ils sont généralement mélangés à un support ("carrier") ou à un agent de charge ("bulking agent") approprié.

Avantageusement, les supports ou agents de charge appropriés sont choisis dans le groupe constitué par le polydextrose, l'amidon, les maltodextrines, la cellulose, la méthylcellulose, la carboxyméthylcellulose et autres dérivés de la cellulose, l'alginate de sodium, les pectines, les gommes, le lactose, le maltose, le glucose, la leucine, le glycérol, le mannitol, le sorbitol, le bicarbonate de sodium, les acides phosphorique, citrique, tartrique, fumarique, benzoïque, sorbique, propionique, et leurs sels de sodium, potassium et calcium, et leurs équivalents.

Les présents agents édulcorants peuvent, dans un produit comestible, être employés seuls, comme unique agent édulcorant, ou en combinaison avec d'autres agents édulcorants tels que les sucres (saccharose), le sirop de maïs, le fructose, les dérivés dipeptidiques sucrés (aspartame, alitame), la néohespéridine dihydrochalcone, l'isomaltulose hydrogéné, le stévioside, les sucres L, la glycyrrhizine, le xylitol, le sorbitol, le mannitol, l'acésulfame-K, la saccharine et ses sels de sodium, potassium, ammonium

et calcium, l'acide cyclamique et ses sels de sodium, potassium et calcium, le sucralose, la monelline, la thaumatine, et leurs équivalents.

Les composés selon l'invention peuvent être préparés selon les techniques classiquement utilisées pour la synthèse peptidique, notamment par condensation d'un chlorure de l'acide 5,6,7,8-tétrahydro-1-naphtoïque ou de l'acide 1-naphtoïque avec l'alpha-L-glutamyl-5-amino-2-pyridinecarbonitrile.

L'acide 1-naphtoïque est d'origine commerciale et l'acide 5,6,7,8-tétrahydro-1-naphtoïque peut être préparé à partir de l'acide 1-naphtoïque par hydrogénation catalytique en présence d'oxyde de platine suivant le procédé décrit dans J. Med. Chem., 1979, 22, 1336-1340. Les chlorures d'acides ont été préparés par chauffage de l'acide correspondant durant 1 h à 50 °C en solution dans le benzène en présence d'un équivalent de pentachlorure de phosphore puis distillés ensuite sous vide à environ 150 °C.

L'alpha-L-glutamyl-5-amino-2-pyridinecarbonitrile est préparé suivant le procédé décrit dans J. Med. Chem. 1973 16, 163-166. Une solution de 5,5 g (24,4 mmol) d'anhydride de l'acide N-trifluoroacétyl-L-glutamique et de 2,6 g (21,8 mmol) de 2-cyano-5-aminopyridine (préparé selon Khim. Geterotsikl. Soedin., 1974 12, 1645-1648) dans le tétrahydrofurane (20 $cm^3$) est agitée durant 12 h à 40 °C. Le solvant est ensuite éliminé et le résidu est dissous dans une solution de carbonate de sodium à 1 % (80 $cm^3$). Le solution est rapidement lavée avec du chlorure de méthylène (3 x 30 $cm^3$) puis est acidifiée à pH 2-3 par une solution de HCl 6N. L'extraction par une solution d'acétate d'éthyle (3 x 50 $cm^3$), suivie d'un séchage sur sulfate de sodium puis de l'évaporation du solvant, conduit à l'obtention de 5,1 g d'un produit brut, le N-trifluoroacétyl-alpha-L-glutamyl-5-amino-2-pyridinecarbonitrile qui est obtenu pur (point de fusion 157 °C) après plusieurs recristallisations dans un mélange éthanolhexane (40-60). Après traitement de ce composé par une solution d'ammoniac à 7,5 % durant 4 h à 20 °C suivi d'une concentration à sec, l'alpha-L-glutamyl-5-amino-2-pyridinecarbonitrile est finalement obtenu (rendement quantitatif, point de fusion 152 °C).

Le N-5,6,7,8-tétrahydro-1-naphtoyl-alpha-L-glutamyl-5-amino-2-pyridinecarbonitrile (formule 6) a été obtenu de la façon suivante :

1,91 g (0,009 mole) de chlorure de 5,6,7,8-tétrahydro-1-naphtoyle, en solution dans le tétrahydrofurane anhydre (50 $cm^3$), est ajouté goutte à goutte à une solution de 1,5 g (0,0064 mole) d'alpha-L-glutamyl-5-amino-2-pyridinecarbonitrile et de 5,05 g (0,06 mole) de $NaHCO_3$ dans 50 $cm^3$ d'eau. Après 15 minutes d'agitation à 20 °C, le tétrahydrofurane est éliminé sous vide et la solution aqueuse restante est acidifiée à pH 2-3 par une solution de HCl 6N, ce qui permet d'obtenir, après filtration et trituration dans de l'hexane, un précipité de 1,4 g de N-5,6,7,8-tétrahydro-1-naphtoyl-alpha-L-glutamyl-5-amino-2-pyridinecarbonitrile (rendement 55 %, point de fusion 141 °C à l'état amorphe). Le pouvoir édulcorant de ce composé est approximativement, sur une base pondérale, 40 000 (quarante mille) fois celui du saccharose par comparaison avec une solution de saccharose à 2 %, 30 000 (trente mille) fois par comparaison avec une solution de saccharose à 5 % et 20 000 (vingt mille) fois par comparaison avec une solution de saccharose à 10 %. Ceci revient à dire qu'une solution aqueuse de 5 mg/L du composé présente une saveur sucrée intense qui est équivalente à celle d'une solution de saccharose à 10 %, ce qui correspond aux intensités édulcorantes généralement utilisées dans les préparations alimentaires.

La préparation du N-1-naphtoyl-alpha-L-glutamyl-5-amino-2-pyridinecarbonitrile (formule 7) est réalisée suivant le même protocole que celui décrit précédemment à partir du chlorure de 1-naphtoyle et d'alpha-L-glutamyl-5-amino-2-pyridinecarbonitrile (rendement 61 %, point de fusion 156 °C à l'état amorphe). Le pouvoir édulcorant de ce composé est approximativement, sur une base pondérale, 30 000 (trente mille) fois celui du saccharose par comparaison avec une solution de saccharose à 2 %, 22 000 (vingt-deux mille) fois par comparaison avec une solution de saccharose à 5 % et 17 000 (dix-sept mille) fois par comparaison avec une solution de saccharose à 10 %. Ceci revient à dire que, dans ces conditions, une solution aqueuse de 5,9 mg/L du composé présente une saveur sucrée intense équivalente à celle d'une solution de saccharose à 10 %, ce qui correspond aux intensités édulcorantes généralement utilisées dans les préparations alimentaires.

## Revendications

1. Agents édulcorants de formule :

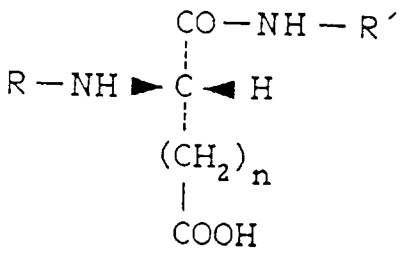

dans laquelle R est un radical 5,6,7,8-tétrahydro-1-naphtoyle de formule :

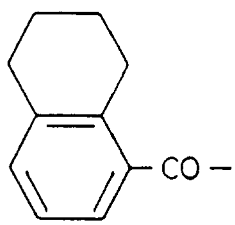

ou un radical 1-naphtoyle de formule :

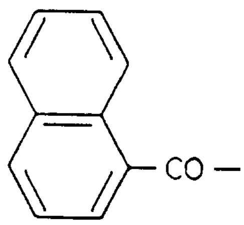

dans laquelle R' est un radical 2-cyanopyrid-5-yle de formule :

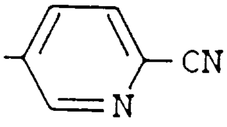

et dans laquelle n est égal à 2.

**2.** Agent édulcorant de formule :

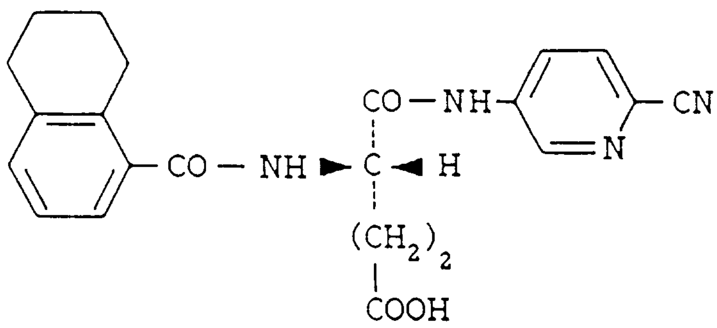

**3.** Agent édulcorant de formule :

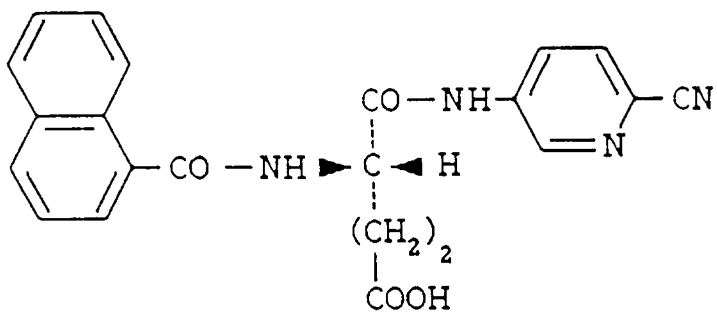

**4.** Procédé de préparation d'un agent édulcorant, de formule :

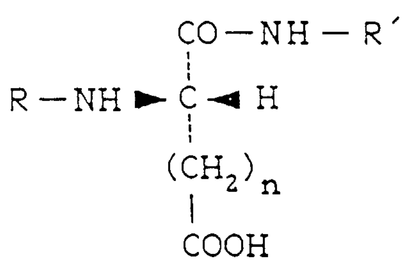

dans laquelle R est un radical 5,6,7,8-tétrahydro-1-naphtoyle de formule :

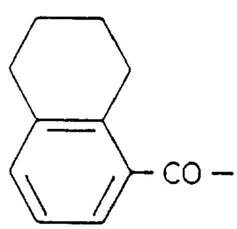

ou un radical 1-naphtoyle de formule :

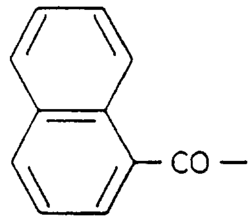

dans laquelle R' est un radical 2-cyanopyrid-5-yle de formule :

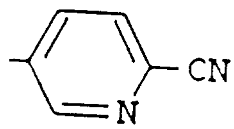

et dans laquelle n est égal à 2,
caractérisé en ce qu'il comprend la condensation d'un chlorure de l'acide 5,6,7,8-tétrahydro-1-naphtoïque ou de l'acide 1-naphtoïque avec l'alpha-L-glutamyl-5-amino-2-pyridinecarbonitrile.

## Claims

**1.** Sweetening agents of the formula

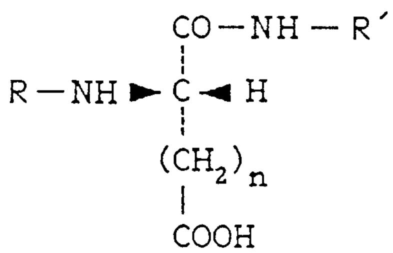

in which R is a 5,6,7,8-tetrahydro-1-naphthoyl radical of the formula

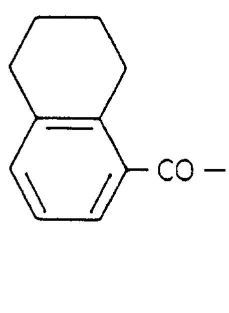

or a 1-naphthoyl radical of the formula

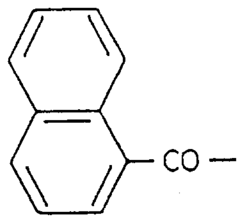

R' is a 2-cyanopyrid-5-yl radical of the formula

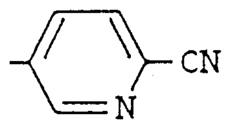

and n is equal to 2.

**2.** Sweetening agent of the formula

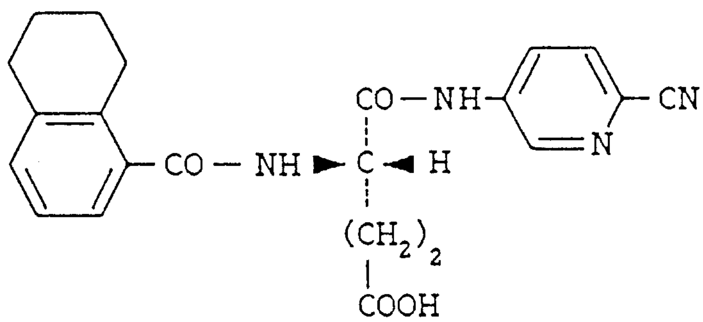

**3.** Sweetening agent of the formula

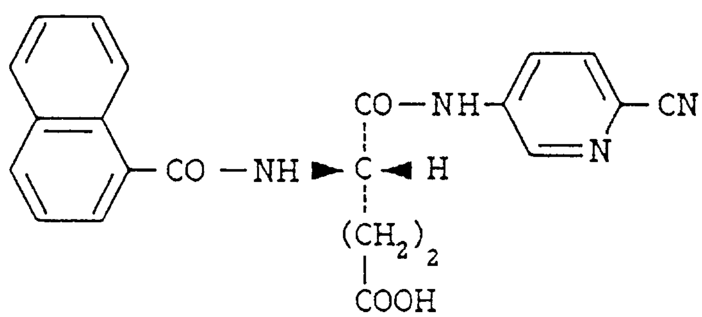

4. Method of preparing a sweetening agent of the formula

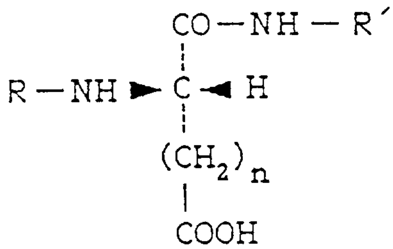

in which R is a 5,6,7,8-tetrahydro-1-naphthoyl radical of the formula

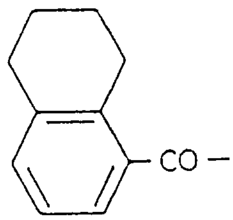

or a 1-naphthoyl radical of the formula

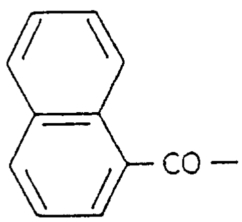

R' is a 2-cyanopyrid-5-yl radical of the formula

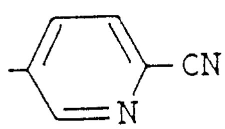

and n is equal to 2,
characterized in that it comprises condensing 5,6,7,8-tetrahydro-1-naphthoyl or 1-naphthoyl acid chloride with alpha-L-glutamyl-5-aminopyridine-2-carbonitrile.

## Patentansprüche

1. Süßstoffe der Formel

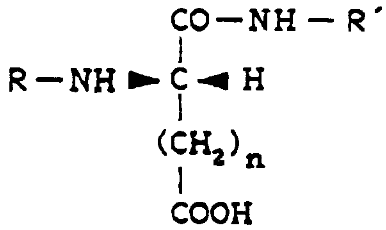

worin R ein 5,6,7,8-Tetrahydro-1-naphthoylrest der Formel ist

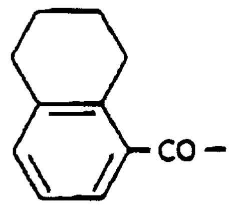

oder ein 1-Naphthoylrest der Formel

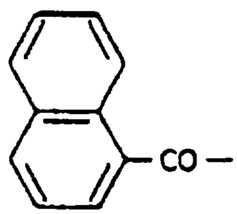

worin R' ein 2-Cyanopyrid-5-ylrest der Formel ist

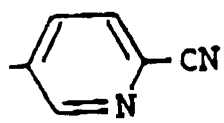

und worin n 2 ist.

2. Süßstoff der Formel

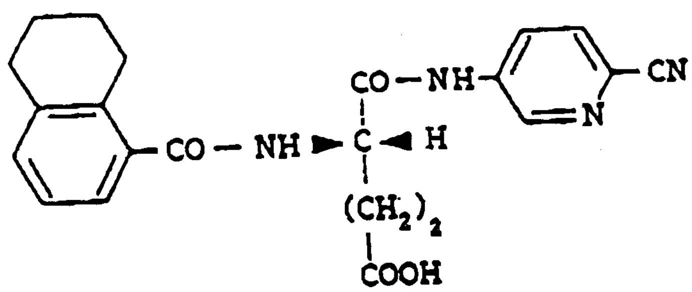

**3.** Süßstoff der Formel

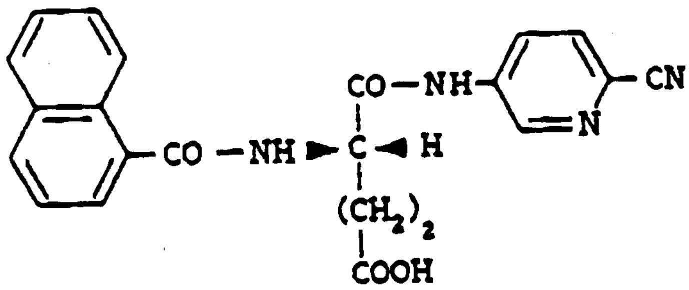

**4.** Verfahren zur Herstellung eines Süßstoffs der Formel

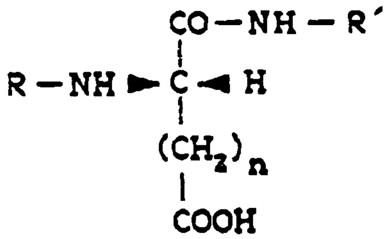

worin R ein 5,6,7,8-Tetrahydro-1-naphthoylrest der Formel ist

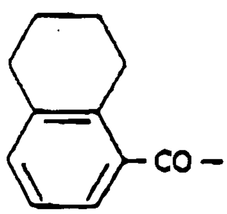

oder ein 1-Naphthoylrest der Formel

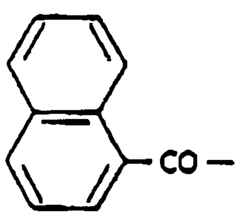

worin R' ein 2-Cyanopyrid-5-ylrest der Formel ist

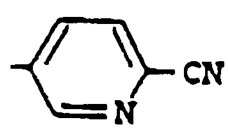

und worin n 2 ist,
dadurch gekennzeichnet, daß es die Kondensation des Chlorids der 5,6,7,8-Tetrahydro-1-naphthoësäure oder der 1-Naphtoësäure mit α-L-Glutamyl-5-amino-2-pyridincarbonitril umfaßt.

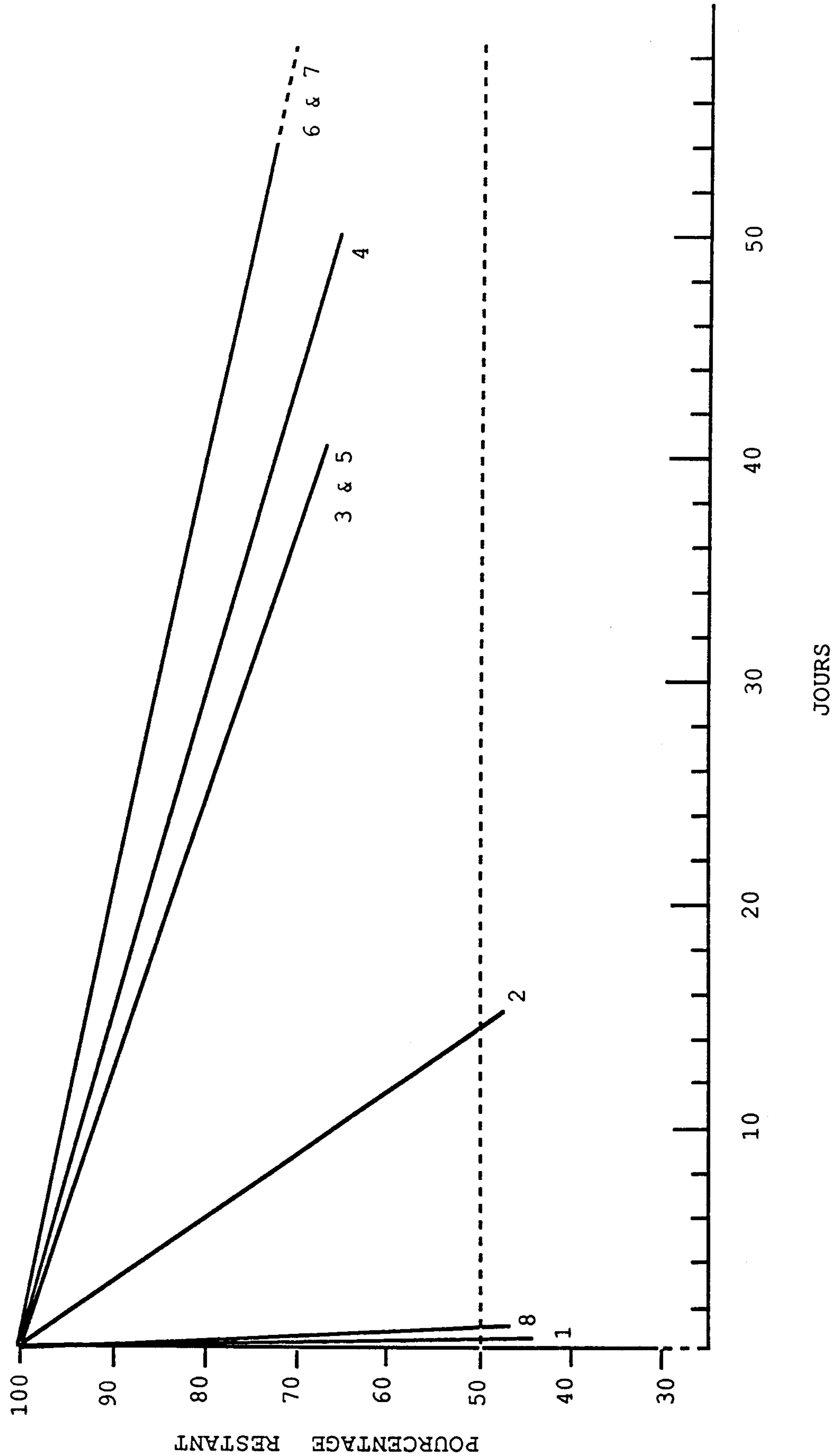


FIGURE 1 : STABILITE COMPAREE (pH 3, 70 °C)